# EUROPEAN PATENT SPECIFICATION

(11) **EP 4 088 695 B1**
(45) Date of publication and mention of the grant of the patent: **07.08.2024**
(21) Application number: 22178366.5
(22) Date of filing: 03.03.2014
(51) Int. Cl.: A61F 9/008

(54) **SYSTEMS FOR PROVIDING ANATOMICAL FLAP CENTRATION FOR AN OPHTHALMIC LASER TREATMENT SYSTEM**
SYSTEME FUR ANATOMISCHE FLAPZENTRIERUNG FÜR EIN AUGENLASERBEHANDLUNGSSYSTEM
SYSTÈMES POUR PRODUIRE UN CENTRAGE DE VOLET ANATOMIQUE POUR UN SYSTÈME DE TRAITEMENT LASER OPHTALMIQUE

(30) Priority: 15.03.2013 US 201361789664 P
(43) Date of publication of application: 16.11.2022
(62) Divisional of application: 14713648.5
(73) Proprietor: AMO Development, LLC, Irvine, CA 92618 (US)
(72) Inventor: BROWNELL, Michael, F., California, 92673 (US); PATTERSON, Kimberley, N., California, 92679 (US); DE GUZMAN, Peter-Patrick, California, 92867 (US); KARDOS, Victor, J., California, 92602 (US); LEE HON, M, California, 92694 (US)
(74) Representative: Carpmaels & Ransford LLP

(56) References cited:
- WO-A1-2010/112041
- WO-A2-2009/033111
- US-A1- 2009 069 794
- US-A1- 2009 247 997
- US-A1- 2011 187 995

## Description

### TECHNICAL FIELD

Embodiments of this invention generally relate to ophthalmic laser surgery, and more particularly, to systems for providing an anatomical flap centration for laser-assisted ophthalmic surgery.

### BACKGROUND

Significant developments in laser technology have led to its application in the field of ophthalmic surgery. In particular, laser surgery has become the technique of choice for ophthalmic procedures where eye tissue is to be photodisrupted or ablated. One particularly popular procedure is laser-assisted in situ keratomileusis ("LASIK"), which is a refractive surgery that treats the cornea of the eye to correct myopia, hyperopia, and/or astigmatism. Since this procedure involves treatment within corneal tissue, it requires a "flap" to be created and lifted to expose a middle section of the cornea (the "stroma") for photoablation with an excimer laser. Previously, surgical tools such as microkeratomes were used to create corneal flaps. But, these days, flaps are created and prepared using a non-ultraviolet, ultra-short pulsed laser that emits radiation in ultra-short pulse durations measured in as few as a few femtoseconds or a few nanoseconds. Examples of ultra-short pulsed laser systems include the Abbott Medical Optics iFS^{™} Advanced Femtosecond Laser, the IntraLase^{™} FS Laser, as well as various other femtosecond and picosecond lasers available in the market.

Laser eye surgery is performed while the patient is in a reclined position but awake, meaning that the patient's eyes are moving during the procedure. As would be expected, patient eye movement relative to the laser beam's focal point can undermine the laser's accuracy and precision, and may even result in permanent tissue damage. For example, when creating flaps, it is extremely importation for the laser beam to accurately focus on a specific focal spot within the corneal tissue. Hence, various devices and mechanisms are conventionally used to stabilize, reduce, and/or eliminate patient eye movement, which in turn, improves safety and surgical outcome.

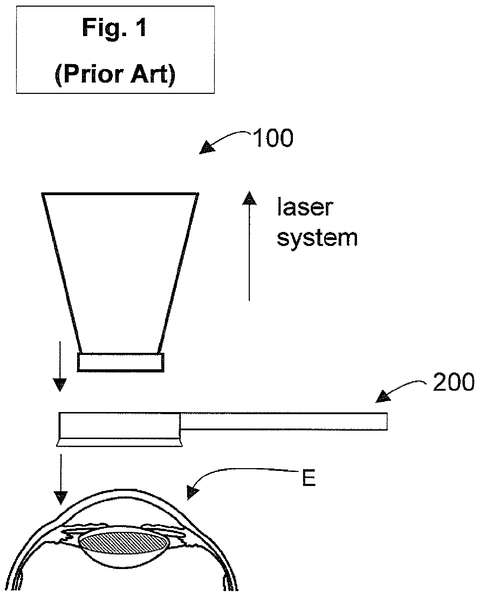

For ultra-short pulsed laser systems, as illustrated in **Fig. 1****,** it is common to use a mechanical stabilization device 100 that directly couples a patient's eye E to the laser's delivery system, thereby limiting the eye's movement. Such a device 100 is commonly referred to as an ophthalmic "patient interface." Examples of ophthalmic patient interface devices used to stabilize the eye are described in U.S. Pat. Nos. 6, 253,476 and 6,254,595, issued to Juhasz et al., U.S. Pat. No. 6, 863, 667, issued to Webb et al., U.S. Pat. No. D462,442 issued to Webb*,* and co-pending U.S. Pat. App. No. 13/230,590. In some systems, the patient interface is held over the anterior surface of the eye using a "suction ring" 200, which is typically the first device secured to the eye. As one can appreciate, even the placement of suction ring 200 and the patient interface 100 prior to surgery requires precision and accuracy. Generally, with conventional systems, surgeons visually estimate the proper placement of the suction ring, the patient interface, as well as the location of the flap, using a digital microscope for guidance. These visual estimations and discretion may leave room for error, leading to less than ideal conditions for alignment of the corneal flap bed, and for the subsequent corrective treatment with the excimer laser. To compensate for the possibility of such error, flaps are commonly oversized. Further prior art is disclosed by WO2010/112041 and US2009/247997.

Hence, there is a need for improved systems for flap placement.

### SUMMARY OF THE INVENTION

Accordingly, embodiments of this invention are directed to systems for providing anatomical flap centration in an ophthalmic laser treatment system, that substantially obviate one or more problems due to limitations and disadvantages of the related art. To achieve these objectives and other advantages, in one embodiment of this invention a surgical laser system, having an imaging system and a suction ring coupled with a patient interface, captures a digital image of the eye and identifies an optimum placement of the flap using anatomical markers as reference points.

This summary and the following detailed description are merely exemplary, illustrative, and explanatory, and are not intended to limit, but to provide further explanation of the invention as claimed. Additional features and advantages of the invention will be set forth in the descriptions that follow, and in part will be apparent from the description, or may be learned by practice of the invention. The objectives and other advantages of the invention will be realized and attained by the structure particularly pointed out in the written description, claims and the appended drawings.

### BRIEF DESCRIPTION OF THE DRAWINGS

Understanding this invention will be facilitated by the following detailed description of the preferred embodiments considered in conjunction with the accompanying drawings, in which like numerals refer to like parts. Note, however, that the drawings are not drawn to scale.
**FIG. 1** is an illustration of an exploded, perspective view of the component portions of an ophthalmic stabilization device known in the art.
**FIG. 2** is a perspective view of a laser eye surgery system according to a preferred embodiment of this invention.
**FIG.** 3 is a simplified diagram of a computer system according to a preferred embodiment of this invention.
**FIG. 4** is an illustration of an electronic process according to a preferred embodiment this invention,
**FIGS. 5a, 5b****,** **5c** are illustrations of the operation of a preferred electronic process and system according to a preferred embodiment of this invention.

### DETAILED DESCRIPTION

The drawings and related descriptions of the embodiments have been simplified to illustrate elements that are relevant for a clear understanding of these embodiments, while eliminating various other elements found in conventional collagen shields, ophthalmic patient interfaces, and in laser eye surgical systems. Those of ordinary skill in the art may thus recognize that other elements and/or steps are desirable and/or required in implementing the embodiments that are claimed and described. But, because those other elements and steps are well known in the art, and because they do not necessarily facilitate a better understanding of the embodiments, they are not discussed. This disclosure is directed to all applicable variations, modifications, changes, and implementations known to those skilled in the art. As such, the following detailed descriptions are merely illustrative and exemplary in nature and are not intended to limit the embodiments of the subject matter or the uses of such embodiments. As used in this application, the terms "exemplary" and "illustrative" mean "serving as an example, instance, or illustration." Any implementation described as exemplary or illustrative is not meant to be construed as preferred or advantageous over other implementations. Further, there is no intention to be bound by any expressed or implied theory presented in the preceding background of the invention, brief summary, or the following detailed description.

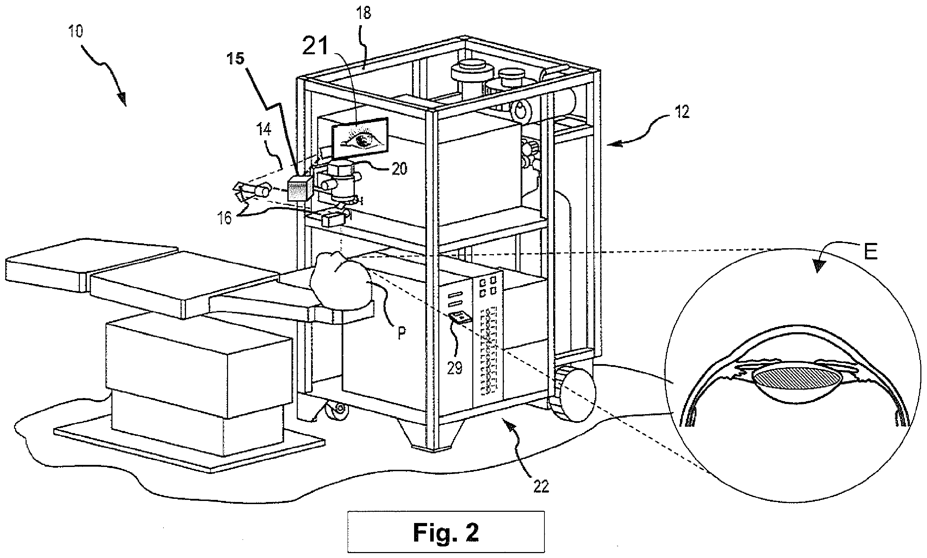

Turning to **Fig. 2****,** an exemplary ophthalmic surgical laser system 10 is shown. The laser system 10 includes a laser 12 that produces a laser beam 14 which generates laser beam pulses. Laser 12 is optically coupled to laser delivery optics 16, which, under the direction of a computer system 22, directs laser beam 14 to an eye E of patient P. A delivery optics support structure (not shown here for clarity) extends from a frame 18 supporting laser 12. A microscope 20 is mounted on the delivery optics support structure. The microscope 20 is generally used by the surgeon during a procedure as guidance to control the system 10 and monitor the status of the patient's eye, E. The surgeon may further use the microscope 20 as a guide to directly engage the eye, e.g., to place a patient interface 100 (as shown in Fig. 1) on the eye or directly treat the eye, if necessary. Preferably, microscope 20 is a digital microscope known in the art that uses, e.g., optics and an imaging device, such as for example, a charged-coupled device ("CCD") camera, to output a digital image to a monitor, such as an LCD display 21. The digital microscope 20 may operate under the direction of the operator and/or the computer system 22. A fixation system 15 is generally coupled to laser 12, laser delivery optics 16 and the delivery optics support structure. The fixation system 15 includes a light emitting diode (LED), optically positioned in front of or above the patient's eye, E, causing the patient to focus on the light when powered on, thereby stabilizing eye movement. The fixation system 15 may be manually manipulated by the surgeon and/or under the direction of computer system 22.

U.S. Patent No. 7,351,241 describes methods of photoalteration. Other devices or systems may also be used to generate pulsed laser beam 14. For example, non-ultraviolet (UV), ultra-short pulsed laser technology can produce pulsed laser beam 14 having pulse durations measured in femtoseconds. Some of the non-UV, ultra-short pulsed laser technology may be used in ophthalmic applications. For example, U.S. Pat. No. 5,993,438 discloses a device for performing ophthalmic surgical procedures to effect high-accuracy corrections of optical aberrations, and also discloses an intrastromal photodisruption technique for reshaping the cornea using a non-UV, ultra-short (e.g., femtosecond pulse duration), pulsed laser beam that propagates through corneal tissue and is focused at a point below the surface of the cornea to photodisrupt stromal tissue at the focal point.

Although the laser system 10 may be used to photoalter a variety of materials (e.g., organic, inorganic, or a combination thereof), the laser system 10 is suitable for ophthalmic applications in one embodiment. In this case, the focusing optics direct the pulsed laser beam 14 toward an eye E (e.g., onto or into a cornea) for plasma mediated (e.g., non-UV) photoablation of superficial tissue, or into the stroma of the cornea for intrastromal photodisruption of tissue. In this embodiment, the laser system 10 may also include a patient interface 100 (as shown in Fig. 1) with a lens to change the shape (e.g., flatten or curve) of the cornea prior to scanning the pulsed laser beam 14 toward the eye E. In this embodiment, the laser system may also use a suction ring 200 in conjunction with the patient interface 100. This suction ring first attaches to the anterior surface of a patient's eye through an applied vacuum mechanism. The laser delivery system 10 along with the patient interface 100 is then guided by the surgeon to subsequently engage with the suction ring. The laser system 10 is capable of generating the pulsed laser beam 14 with physical characteristics similar to those of the laser beams generated by a laser system disclosed in U.S. Pat. No. 4,764,930 and U.S. Pat. No. 5, 993, 438.

For example, the ophthalmic laser system 10 can produce an ultra-short pulsed laser beam 14 for use as an incising laser beam 14. This pulsed laser beam 14 preferably has laser pulses with durations as long as a few nanoseconds or as short as a few femtoseconds. For intrastromal photodisruption of the tissue, the pulsed laser beam 14 has a wavelength that permits the pulsed laser beam 14 to pass through the cornea without absorption by the corneal tissue. The wavelength of the pulsed laser beam 14 is generally in the range of about 400 nm to about 3000 nm, and the irradiance of the pulsed laser beam 14 for accomplishing photodisruption of stromal tissues at the focal point is typically greater than the threshold for optical breakdown of the tissue. Although a non-UV, ultra-short pulsed laser beam is described in this embodiment, the pulsed laser beam may have other pulse durations and different wavelengths in other embodiments.

Computer system 22 may comprise (or interface with) a conventional of special computer, e.g., PC, laptop, and so on, including the standard user interface devices such as a keyboard, a mouse, a touch pad, foot pedals, a joystick, a touch screen, an audio input, a display monitor, and the like. Computer system 22 typically includes an input device such as a magnetic or optical disk drive, or an input interface such as a USB connection, a wired and/or wireless network connection, or the like. Such input devices or interfaces are often used to download a computer executable code, to a storage media 29, and may embody any of the methods according to embodiments of this invention. Storage media 29 may take the form of an optical disk, a data tape, a volatile or non-volatile memory, RAM, or the like, and the computer system 22 includes the memory and other standard components of modern computer systems for storing and executing this code. Storage media 29 includes one or more fixation maps, and may optionally include a treatment map, and/or an ablation table. Storage media 29 may alternatively be remotely operatively coupled with computer system 22 via network connections such as LAN, the Internet, or via wireless methods such as WLAN, Bluetooth, or the like.

Additional components and subsystems may be included with laser system 10, as should be understood by those of skill in the art. For example, spatial and/or temporal integrators may be included to control the distribution of energy within the laser beam, as described in U.S. Patent No. 5,646,791. Ablation effluent evacuators/filters, aspirators, and other ancillary components of the laser surgery system are known in the art. Further details of suitable systems for performing a laser ablation procedure can be found in commonly assigned U.S. Pat. Nos. 4,665,913, 4,669,466, 4,732,148, 4,770,172, 4,773,414, 5,207,668, 5,108,388, 5,219,343, 5,646,791 and 5,163,934.

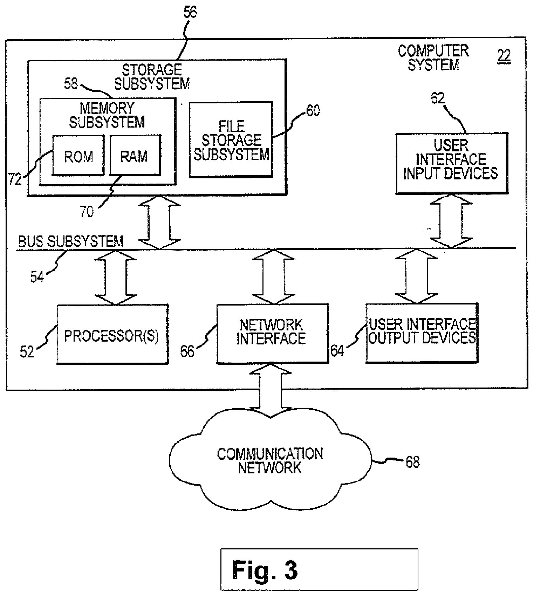

**Fig. 3** is a simplified block diagram of an exemplary computer system 22 that may be used by the laser surgical system 10 of embodiments of this invention. Computer system 22 typically includes at least one processor 52 which may communicate with a number of peripheral devices via a bus subsystem 54. These peripheral devices may include a storage subsystem 56, comprising a memory subsystem 58 and a file storage subsystem 60 (which may include storage media 29), user interface input devices 62, user interface output devices 64, and a network interface subsystem 66. Network interface subsystem 66 provides an interface to outside networks 68 and/or other devices.

User interface input devices 62 may include a keyboard, pointing devices such as a mouse, trackball, touch pad, or graphics tablet, a scanner, foot pedals, a joystick, a touch screen incorporated into the display, audio input devices such as voice recognition systems, microphones, and other types of input devices. User interface input devices 62 are often used to download a computer executable code from a storage media 29 embodying any of the methods according to embodiments of this invention. User interface input devices 62 are also used to control an eye fixation system. In general, use of the term "input device" is intended to include a variety of conventional and proprietary devices and ways to input information into computer system 22.

User interface output devices 64 may include a display subsystem, a printer, a fax machine, or non-visual displays such as audio output devices. The display subsystem may be a cathode ray tube (CRT), a flat-panel device such as a liquid crystal display (LCD), e.g., LCD display 21 shown in Fig. 2, a projection device, or the like. The display subsystem may also provide a non-visual display such as via audio output devices. In general, use of the term "output device" is intended to include a variety of conventional and proprietary devices and ways to output information from computer system 22 to a user.

Storage subsystem 56 can store the basic programming and data constructs that provide the functionality of the various embodiments of this invention. For example, a database and modules implementing the functionality of the methods described here may be stored in storage subsystem 56. These software modules are generally executed by processor 52. In a distributed environment, the software modules may be stored on a plurality of computer systems and executed by processors of the plurality of computer systems. Storage subsystem 56 typically comprises memory subsystem 58 and file storage subsystem 60.

Memory subsystem 58 typically includes a number of memories including a main random access memory (RAM) 70 for storage of instructions and data during program execution and a read only memory (ROM) 72 in which fixed instructions are stored. File storage subsystem 60 provides persistent (non-volatile) storage for program and data files, and may include storage media 29 (Fig. 2). File storage subsystem 60 may include a hard disk drive along with associated removable media, a Compact Disk (CD) drive, an optical drive, DVD, solid-state removable memory, and/or other removable media cartridges or disks. One or more of the drives may be located at remote locations on other connected computers at other sites coupled to computer system 22. The modules implementing the functionality of embodiments of this invention may be stored by file storage subsystem 60.

Bus subsystem 54 provides a mechanism for letting the various components and subsystems of computer system 22 communicate with each other as intended. The various subsystems and components of computer system 22 need not be at the same physical location but may be distributed at various locations within a distributed network. Although bus subsystem 54 is shown schematically as a single bus, alternate embodiments of the bus subsystem may utilize multiple busses.

Computer system 22 itself can be of varying types including a personal computer, a portable computer, a workstation, a computer terminal, a network computer, a control system in a wavefront measurement system or laser surgical system, a mainframe, or any other data processing system. Due to the ever-changing nature of computers and networks, the description of computer system 22 depicted in **Fig. 2** is intended only as an example for purposes of illustrating one embodiment of this invention. Many other configurations of computer system 22, having more or fewer components than the computer system depicted in **Fig. 3****,** are possible.

In the current state of the art, when physicians place suction ring 200 on the eye E, they will visually estimate the centration of the suction ring 200 on the eye, E. Once the suction ring 200 is applied to the eye, the patient interface 100 is brought down, visually centered to the eye E, and then mechanically engaged to the suction ring 200 using vacuum pressure. Because the suction ring is the first device attached onto the eye, its placement defines the centration of the patient interface 100 over the eye E. Surgeons may thus visually estimate the centration of the flap using the patient interface 100 as guidance. Not only may this discretion leave room for error, but any error in the placement of the suction ring 200 and patient interface 100 will compound the error (if any) in flap placement.

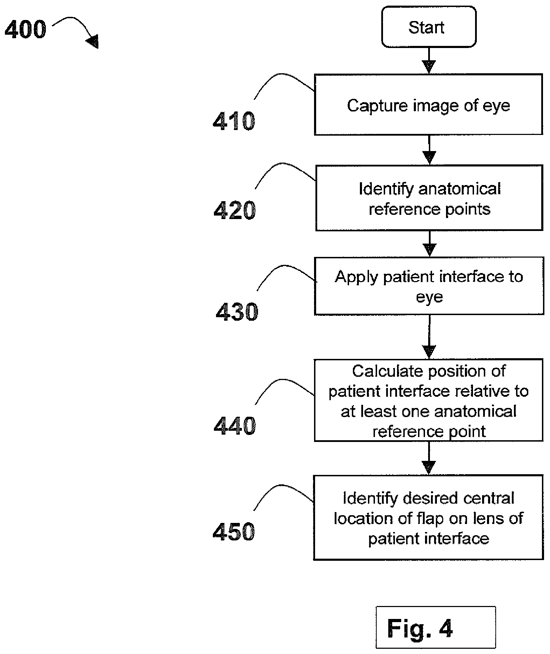

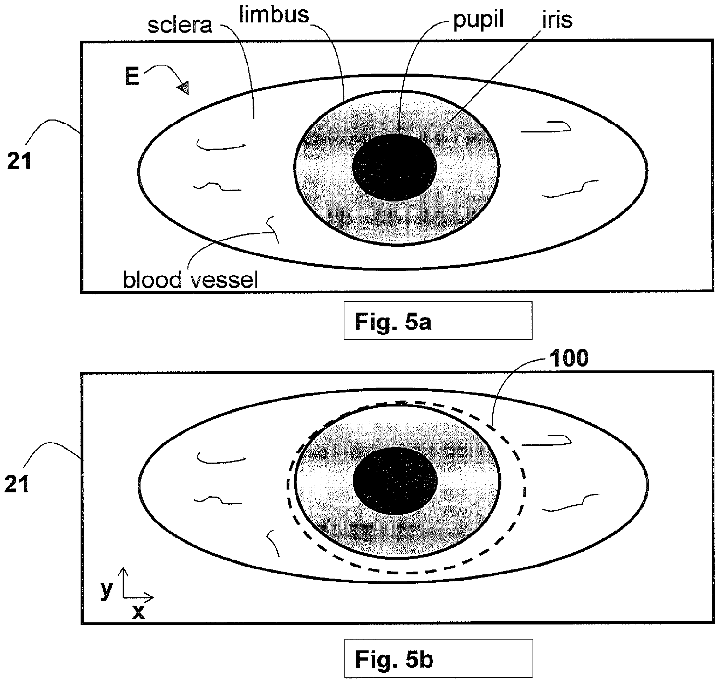

Turning to **Fig. 4****,** an electronic process 400 to address this issue is shown. Using digital microscope 20, an image of the eye, E is captured (Action Block 410). Turning to **Fig. 5a****,** an exemplary image of eye, E, is shown on LCD Display 21. Using an image processor and image recognition software known in the art, included, e.g., in computer system 22, the location of certain anatomical reference points may be calculated, e.g., the location of the limbus, sclera, pupil, iris and blood vessels (Action Block 420). Concurrently, features on the suction ring 200 (since it defines patient interface placement) can also be tracked and referenced. From these reference points, the optimum location for the flap can be identified, such as, for example, over the center of the pupil. These reference points can also be used to subsequently align the excimer laser for photoablation. More detail about the image processing, recognition, identification, and centration steps can be found in U.S. Patent Nos. 5,966,197; 6,283,954; 6367,931; 7431,457; and 7,480,396.

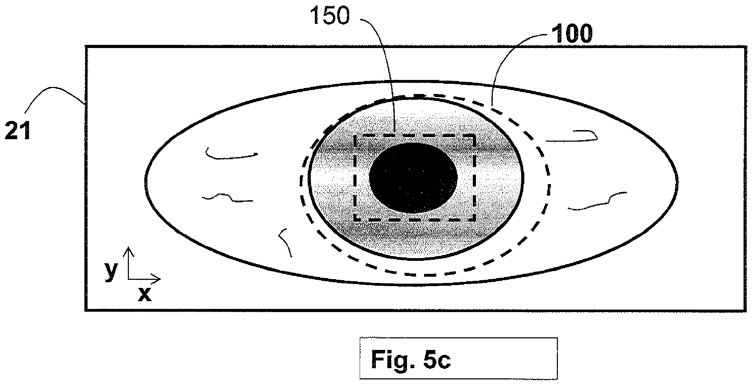

Next, the surgeon may attach the suction ring 200 and patient interface 100 to the eye E (Action Block 430). He or she may visually estimate the centration of the suction ring 200 and the patient interface 100 using digital microscope 20 and digital display 21. This visual estimation may lead to an imperfect centration. Turning to **Fig. 5b****,** an imperfectly-centrated patient interface 100 is shown. Using the image processor and imaging recognition software in computer system 22, the imperfection can be calculated in terms of the suction ring 200 (x,y) displacement relative to the previously located anatomical references, for example, the pupil, limbus, etc. (*See* Action Block 440 in **Fig. 4**). Instead, of centrating the flap based solely on the suction ring 200 and patient interface 100, the (x,y) displacement may also be incorporated into the flap centration calculation. Thus, the flap is more precisely centrated on the eye E, based on the location of the anatomical reference points (*See* Action Block 450 in **Fig. 4**). Turning to Fig. **5c****,** the image processor in computer system 22 may create an overlay 150 on the image of the eye E on LCD Display 21 marking an ideal (*e.g*., precisely and accurately centrated) flap location. This overlay 150 may be reviewed and approved by the surgeon prior to actual delivery of laser energy onto the eye E.

In an alternative embodiment, image processor in computer system 22 can also create an overlay on LCD display 21 for an optimum placement of the suction ring 200 and patient interface 100 prior to creating overlay of flap location 150, enabling the surgeon to adjust any error in placement of the suction ring 200 and patient interface 100.

In an alternative embodiment, a set of sensors, such as for example, accelerometers, gyroscopes, or magnetometers, may be mechanically registered or affixed to the suction ring 200 to provide direct spatial information based on the appropriate reference. The spatial information, in conjunction with the image processing for tracking of anatomical features on the eye, will provide appropriate positioning guidance and error tracking to minimize centration error. The addition of sensors also provides redundant information to the image processing performed to track the suction ring. This improves overall robustness of the centration system since a cross-checking algorithm can be implemented to ensure data integrity.

In an alternative embodiment, structured light can be implemented to provide the appropriate Purkinje reflections for image processing to provide information on the orientation and position of the visual axis as well as the apex of the cornea. The visual axis orientation and corneal apex is then linked to the anatomical features (*e.g.* pupil, limbus, iris, blood vessels, *etc*.) of the eye to enable reference after flap creation. This data set, in conjunction with the suction ring reference, can also provide positioning guidance and error tracking to minimize centration error.

Although embodiments of this invention are described and pictured in an exemplary form with a certain degree of particularity, describing the best mode contemplated of carrying out the invention, and of the manner and process of making and using it, those skilled in the art will understand that various modifications, alternative constructions, changes, and variations can be made in the ophthalmic interface and method without departing from the spirit or scope of the invention. As a specific example, any appropriate laser medium might be used to deliver the incident laser beam without regard to the particular form and shape of the delivery system. Likewise, any type of image capturing, processing and recognition systems may be used, without departing from the scope and spirit of the invention. Thus, it is intended that this invention cover all modifications, alternative constructions, changes, variations, as well as the combinations and arrangements of parts, structures, and steps that come within the scope of the invention as defined by the following claims.

## Claims

1. An ophthalmic surgical laser system (10) for creating a corneal flap on a subject's eye (E),comprising:
a laser delivery system (12);
a suction ring (200) configured to overlay an anterior surface of the subject's eye, the suction ring operatively coupled to a patient interface (100), wherein the patient interface is coupled to the laser delivery system;
an imaging system operatively coupled to the laser delivery system and configured to capture a digital image of the subject's eye with the suction ring overlaying the anterior surfaceof the eye, wherein the digital image contains one or more Purkinje reflections of structured light;
a computer-readable medium having a set of instructions that, when executed by a processor (52), identifies an optimum location for creating the corneal flap, wherein the set of instructions comprise:
identifying one or more anatomical reference points in digital image captured bythe imaging system;
based on the Purkinje reflections, generating a data set of information on the orientation and position of the visual axis as well as the apex of the cornea;
linking the visual axis orientation and corneal apex to the anatomical reference points;
calculating the location of the suction ring coupled to the patient interface relative to the reference points; and
identifying a location on the cornea to create the flap based on the location of the suction ring coupled to the patient interface relative to the reference points on the cornea and the data set.

2. The system of claim 1, wherein the set of instructions includes centrating the location on the cornea to create the flap based on the location of the suction ring coupled to the patient interface relative to the one or more reference points on the cornea.

3. The system of claim 1, wherein the laser delivery system is a ultra-short pulsed lasersystem.

4. The system of claim 1, wherein the set of instructions includes generating an overlay on the image that identifies the central location on the cornea to create the flap based on the locationof the suction ring coupled to the patient interface relative to the one or more reference points on the cornea.

5. The system of claim 1, wherein the set of instructions includes generating an overlay on the image that identifies the central location on the cornea to place the suction ring coupled to thepatient interface based on the one or more reference points on the cornea.

6. The system of claim 1, wherein the one or more reference points includes the location of at least one of a pupil, a limbus, an iris, a sclera, and a blood vessel of the subject's eye.

7. The system of claim 1, wherein the data set is used in conjunction with the location of the suction ring coupled to the patient interface relative to the reference points to provide positioning guidance and error tracking to minimize centration error.

## Patentansprüche

1. Lasersystem für die Augenchirurgie (10) zum Erzeugen eines Hornhaut-Flaps auf einem Auge (E) eines Subjekts, umfassend:
ein Laserabgabesystem (12);
einen Saugring (200), der ausgestaltet ist, um über einer anterioren Oberfläche des Auges des Subjekts zu liegen,
wobei der Saugring funktionell an eine Patientenschnittstelle (100) gekoppelt ist, wobei die Patientenschnittstelle an das Laserabgabesystem gekoppelt ist;
ein Bildgebungssystem, das funktionell an das Laserabgabesystem gekoppelt ist und ausgestaltet ist, um ein digitales Bild des Auges des Subjekts zu erfassen,
bei dem der Saugring über der anterioren Oberfläche des Auges liegt, wobei das digitale Bild eine oder mehrere Purkinje-Reflexionen von strukturiertem Licht enthält;
ein computerlesbares Medium mit einem Satz von Anweisungen, der bei Ausführung durch einen Prozessor (52) eine optimale Stelle zum Erzeugen eines Hornhaut-Flaps identifiziert, wobei der Satz der Anweisungen umfasst:
Identifizieren von einem oder mehreren anatomischen Bezugspunkten in dem digitalen Bild, welches durch das Bildgebungssystem erfasst wurde;
basierend auf den Purkinje-Reflexionen, Generieren eines Datensatzes von Informationen über die Orientierung und Position der Sehachse sowie des Hornhautscheitels;
Verknüpfen der Orientierung der Sehachse und des Hornhautscheitels mit den anatomischen Bezugspunkten;
Berechnen der Stelle des Saugrings, die an die Patientenschnittstelle gekoppelt ist, relativ zu den Bezugspunkten; und
Identifizieren einer Stelle auf der Hornhaut zum Erzeugen des Flaps basierend auf der Stelle des Saugrings, der an die Patientenschnittstelle gekoppelt ist, relativ zu den Bezugspunkten auf der Hornhaut und dem Datensatz.

2. System nach Anspruch 1, wobei der Satz der Anweisungen das Zentrieren der Stelle auf der Hornhaut, um den Flap zu erzeugen, basierend auf der Stelle des Saugrings, der an die Patientenschnittstelle gekoppelt ist, relativ zu dem einen oder den mehreren Bezugspunkten auf der Hornhaut einschließt.

3. System nach Anspruch 1, wobei das Laserabgabesystem ein ultrakurz gepulstes Lasersystem ist.

4. System nach Anspruch 1, wobei der Satz der Anweisungen das Generieren einer Überlagerung auf dem Bild einschließt, welche die zentrale Stelle auf der Hornhaut, um den Flap zu erzeugen, basierend auf der Stelle des Saugrings, der an die Patientenschnittstelle gekoppelt ist, relativ zu dem einen oder den mehreren Bezugspunkten auf der Hornhaut identifiziert.

5. System nach Anspruch 1, wobei der Satz der Anweisungen das Erzeugen einer Überlagerung auf dem Bild einschließt, die die zentrale Stelle auf der Hornhaut identifiziert, um den an die Patientenschnittstelle gekoppelten Saugring basierend auf dem einen oder den mehreren Bezugspunkten auf der Hornhaut zu platzieren.

6. System nach Anspruch 1, wobei der eine oder die mehreren Bezugspunkte die Stelle von mindestens einem von einer Pupille, einem Limbus, einer Iris, einer Sklera und einem Blutgefäß des Auges des Subjekts einschließt bzw. einschließen.

7. System nach Anspruch 1, wobei der Datensatz in Verbindung mit der Stelle des Saugrings, der an die Patientenschnittstelle gekoppelt ist, relativ zu den Bezugspunkten verwendet wird, um eine Positionierungsführung und eine Fehlerverfolgung zur Minimierung von Zentrierungsfehlern bereitzustellen.

## Revendications

1. Système laser chirurgical ophtalmique (10) pour créer un volet cornéen sur l'œil d'un sujet (E), comprenant :
un système de distribution de laser (12) ;
un anneau d'aspiration (200) configuré pour recouvrir une surface antérieure de l'œil du sujet, l'anneau d'aspiration étant couplé de manière opérationnelle à une interface patient (100), l'interface patient étant couplée au système de distribution de laser ;
un système d'imagerie couplé de manière opérationnelle au système de distribution de laser et configuré pour capturer une image numérique de l'œil du sujet avec l'anneau d'aspiration recouvrant la surface antérieure de l'œil, l'image numérique contenant une ou plusieurs réflexions de Purkinje de lumière structurée ;
un support lisible par ordinateur contenant un ensemble d'instructions qui, lorsqu'elles sont exécutées par un processeur (52), identifient un emplacement optimal pour la création du volet cornéen, l'ensemble d'instructions comprenant :
l'identification d'un ou plusieurs points de référence anatomiques dans une image numérique capturée par le système d'imagerie ;
sur la base des réflexions de Purkinje, la génération d'un ensemble d'informations sur l'orientation et la position de l'axe visuel ainsi que sur l'apex de la cornée ;
la liaison de l'orientation de l'axe visuel et de l'apex de la cornée aux points de référence anatomiques ;
le calcul de l'emplacement de l'anneau d'aspiration couplé à l'interface patient par rapport aux points de référence ; et
l'identification d'un emplacement sur la cornée pour créer le volet sur la base de l'emplacement de l'anneau d'aspiration couplé à l'interface patient par rapport aux points de référence sur la cornée et à l'ensemble de données.

2. Système selon la revendication 1, l'ensemble d'instructions comprenant le centrage de l'emplacement sur la cornée pour créer le volet sur la base de l'emplacement de l'anneau d'aspiration couplé à l'interface patient par rapport au ou aux points de référence sur la cornée.

3. Système selon la revendication 1, le système de distribution de laser étant un système de laser à impulsions ultra-courtes.

4. Système selon la revendication 1, l'ensemble d'instructions comprenant la génération d'une superposition sur l'image qui identifie l'emplacement central sur la cornée pour créer le volet sur la base de l'emplacement de l'anneau d'aspiration couplé à l'interface patient par rapport au ou aux points de référence sur la cornée.

5. Système selon la revendication 1, l'ensemble d'instructions comprenant la génération d'une superposition sur l'image qui identifie l'emplacement central sur la cornée pour placer l'anneau d'aspiration couplé à l'interface patient sur la base du ou des points de référence sur la cornée.

6. Système selon la revendication 1, le ou les points de référence comprenant l'emplacement d'au moins un élément parmi : une pupille, un limbe, un iris, une sclérotique et un vaisseau sanguin de l'œil du sujet.

7. Système selon la revendication 1, l'ensemble de données étant utilisé en conjonction avec l'emplacement de l'anneau d'aspiration couplé à l'interface patient par rapport aux points de référence pour fournir un guidage de positionnement et un suivi d'erreur afin de minimiser l'erreur de centrage.
